Europäisches Patentamt

European Patent Office

Office européen des brevets

(19)

(11) Publication number : **0 467 676 A2**

# EUROPEAN PATENT APPLICATION

(12)

(21) Application number : **91306531.4**

(22) Date of filing : **18.07.91**

(51) Int. Cl.$^5$ : **C12N 15/70,** C12N 15/12, C12N 15/31

The applicant has subsequently filed a sequence listing and declared, that it includes no new matter.

(30) Priority : **18.07.90 US 555579**

(43) Date of publication of application :
**22.01.92 Bulletin 92/04**

(84) Designated Contracting States :
**AT BE CH DE DK ES FR GB GR IT LI LU NL SE**

(71) Applicant : **APPLIED IMMUNESCIENCES, INC.
200 Constitution Drive
Menlo Park California 94025-1109 (US)**

(72) Inventor : **Beerman, Nancy
2310 Rock Street
No. 20 Mountain View, California 94043 (US)**
Inventor : **Talib, Sohel
34666 Siward Drive
Fremont, California 94536 (US)**
Inventor : **Okarma, Thomas B.
1651 Portola Avenue
Palo Alto, California 94306 (US)**

(74) Representative : **Goldin, Douglas Michael et al
J.A. KEMP & CO. 14, South Square Gray's Inn
London WC1R 5LX (GB)**

(54) **Temperature-inducible secretion expression vectors and their use in prokaryotic cells.**

(57)     Temperature-inducible secretion expression vectors have been constructed and used to express heterologous proteins in E. coli. The vectors use a promoter comprising a nucleotide capable of binding the $C_l$ repressor to provide for temperature-inducible expression from the promoter. Secretion of the native heterologous protein is achieved by replacing the coding region adjacent to the promoter signal sequence with a DNA sequence encoding the heterologous protein. The secretion expression vector is transformed into a host cell which is then grown and induced to express the heterologous protein. No expression is seen at 30°C, but upon induction at 42°C, protein is produced. Expression levels of 5-10% of total protein are seen. The heterologous protein is shown to be correctly processed and biologically active. These vectors can be used to express and secrete a protein of interest in prokaryotic cells lacking a temperature-sensitive mutation by inserting the appropriate gene in phase with the signal sequence.

EP 0 467 676 A2

Technical Field

This invention relates to compositions and methods for production of polypeptides, particularly heterologous polypeptides, in microorganisms using recombinant DNA techniques.

Background

The rapid development of molecular biology techniques continues to increase the feasibility of cloning genes of interest. So, too, the need for suitable methods of producing large quantities of heterologous proteins for research, therapeutic or industrial purposes increases. The use of E. coli as a host for expression of foreign proteins has become relatively commonplace, and several vectors have been developed for the high-level expression of foreign proteins in E. coli.

Temperature-sensitive vectors provide the advantage of easy induction of expression by simply shifting the culture temperature from 30°C to 42°C. Temperature-inducible expression also allows for the optimization of growth media for maximum yield of heterologous protein without the concern of catabolite repression or feedback inhibition. An additional advantage is that use of a temperature-sensitive vector eliminates the need for Isopropyl ß-D-thiogalactoside (IPTG), commonly used for the induction of expression from vectors that use the lac or tac promoters, which can add to the expense of a large-scale production of protein. There are, however, significant disadvantages with currently available expression vectors for use in E. coli: frequently the protein is found in an insoluble and inactive form in the cytoplasm of the cell. Activity can be restored if the protein can be solubilized during or after purification, but this is a difficult and often unsuccessful process.

Secretion expression vectors have been developed which use signal peptides to transport expressed protein into the periplasmic space of E. coli, where the protein is often found in a soluble, active form. The use of secretion expression vectors provides additional advantages of protection of heterologous protein from proteolytic degradation, tolerated expression of a protein harmful to the host cell, production of a protein with an amino terminus identical to a naturally occurring protein, and simplified purification due to the presence of fewer contaminating proteins in the periplasmic space. It would therefore be of interest to develop secretion expression vectors which combine the advantages of temperature-inducible expression with the advantages of secretion for the production of heterologous proteins in E. coli.

Relevant Literature

A temperature-sensitive vector, pHE6, has been disclosed by Milman, Methods in Enzymology (1987) 153:482-491. Other vectors designed for high-level expression of heterologous proteins in E. coli have been described. Duffard et al., Methods in Enzymology (1987) 153:492:507; Greenfield et al., Biotechnology (1986) 4:1006-1011; Tabor and Richardson, Proc. Natl Acad. Sci (USA) (1988) 82:1074-1078; Crowl et al., Gene (1985) 38:31-38.

Secretion expression vectors have been described by Ghrayeb et al., EMBO J. (1984) 3:2437-2442 and Miyake et al., J. Biochem. (1985) 97:1429-1436. Correct formation of disulfide bonds (Dollitt and Zalkin, J. Bacteriol. (1983) 27-32; and Hsiung et al., Biotechnology (1986) 4:991-995); and of active dimers in the periplasmic space (Skerra and Pluckthun, Science (1988) 240:1038-1041) have also been reported. For a review of secretion vectors, see Marston, Biochem. J. (1986) 240:1-12.

SUMMARY OF THE INVENTION

Secretion expression vectors and methods for their preparation and use are described which provide for temperature-sensitive induction of expression and secretion of an active gene product from a prokaryotic host cell. The expression vectors include a transcriptional initiation region comprising a nucleotide sequence capable of binding to a temperature-sensitive repressor, a DNA sequence encoding a signal sequence capable of directing secretion of a polypeptide of interest from a host cell, and an optional transcriptional termination region. The secretion vector additionally includes a gene encoding a temperature-sensitive repressor. In use, host cells are transformed with the expression vector, after which they are grown in a nutrient medium at a permissive temperature until a desired cell density is reached. The temperature is then increased to a temperature sufficient to inactivate the repressor to induce transcription of said polypeptide of interest.

BRIEF DESCRIPTION OF DRAWINGS

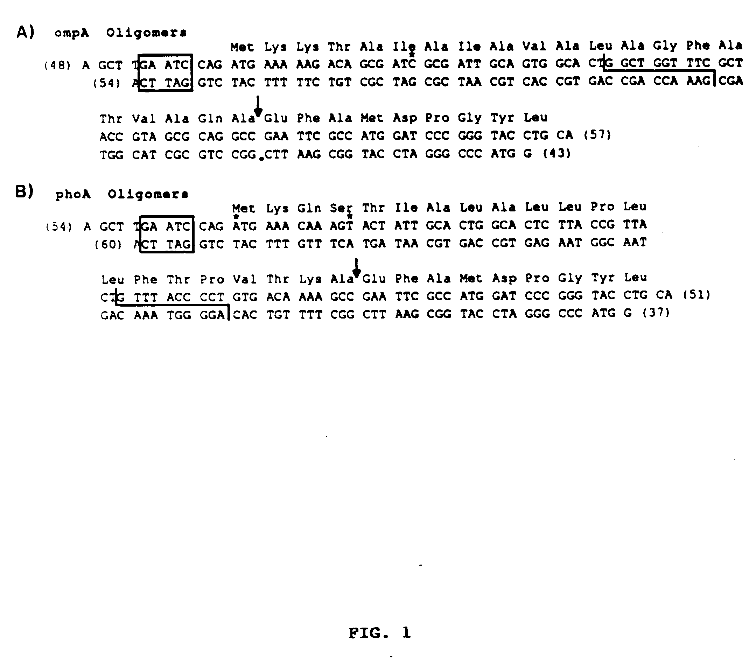

Figure 1 shows the nucleotide sequences of the oligomers used to construct the ompA (A) and phoA (B) synthetic signal sequences of pNB1 and pNB2, respectively: The lines indicate the ends and overlapping regions of the oligomers; the asterisks mark the nucleotide changes from the naturally occurring sequences; the arrows indicate the cleavage sites of the signal peptides; the boxes mark the Hinfl restriction sites used to ligate the synthetic fragments to the naturally occurring Hinfl site at-7 in the lambda N-protein gene and the numbers in parentheses give the sizes of the respective oligomers.

Figure 2 is a diagram for assembly and cloning of synthetic signal sequences.

Figure 3 is a diagram for the construction of pNB1 and pNB2.

Figure 4 shows maps of pNB1 and pNB2.

Figure 5 shows the insertion of αacr in phase with the signal sequences(s) of pNB1 (ompA) and pNB2 (phoA).

Figure 6 shows the results of in vitro coupled transcription-translation of pNB1αacr and pNB2αacr.

Figure 7 shows the results of expression of αacr in vivo.

Figure 8 shows in vitro labeling with $^{35}$S-methionine.

Figure 9 shows the confirmation of protein identity and demonstration of biological activity with α-bungarotoxin.

Figure 10 shows expression of a gene encoding protein A binding regions (SG) and a chimeric gene encoding a protein comprising BG and protein A binding regions using pNB1 and pNB2.

Figure 11 shows SDS-PAGE analysis of periplasmic, spheroplast and total cell extract of the host cells described in Figure 10.

## BRIEF DESCRIPTION OF SPECIFIC EMBODIMENTS

In accordance with the subject invention, temperature-inducible secretion vectors, and methods for their preparation and use, are described. The secretion vectors provide for temperature-inducible expression and secretion of an active gene product. The expression vectors include a temperature-sensitive repressor to control expression from the promoter to provide for temperature-inducible expression of a desired polypeptide and further include a signal sequence for secretion of the polypeptide of interest. The secretion expression vectors are introduced into a host cell under conditions whereby the resulting transformants stably maintain the secretion expression vector. Naturally occurring DNA or synthetic DNA may be employed for the production of a polypeptide of interest encoded by the DNA. The secretion expression vector provides for a fused expression product comprising the signal peptide and the polypeptide of interest.

The secretion expression vectors will have the following basic structure:

$$P\text{--}S.D.\text{--}Met\text{--}S\text{--}G\text{--}(T)_{0 \text{ or } 1}$$

wherein:

P comprises a promoter sequence encoding the regulatory regions occurring at about -35 and -10 nucleotides upstream from the translation start site together with sequences capable of binding to the $C_I$ repressor;

S.D. comprises a Shine-Dalgarno sequence;

Met comprises a codon for the initiating methionine of the signal peptide of interest;

S comprises a DNA sequence encoding a signal sequence allowing for secretion of the polypeptide of interest from the host cell and cleavage of the signal sequence from the polypeptide of interest;

G comprises a DNA sequence encoding the polypeptide of interest; and

T comprises an optional transcriptional termination region.

The promoter or transcriptional regulatory region in prokaryotic cells comprises nucleotide sequences which can affect the efficiency of transcription. The sequences include the regulatory regions at about -35 and -10 nucleotides from the translation start site. The transcriptional regulatory region additionally will include regulatory sequences which allow the time of expression of the DNA sequence encoding the polypeptide of interest to be modulated by the temperature of the growth medium. Illustrative transcriptional regulatory regions or promoters which provide for temperature-sensitive transcription include any promoters which will bind the $C_I857$ repressor (for example, the lambda left and right promoters, and the like). Synthetic promoters having nucleotide sequences at least substantially similar to these sequences may also find use.

A preferred transcriptional regulatory region is the bacteriophage $\lambda P_L$ promoter. When the $\lambda P_L$ promoter is used, the transcriptional regulatory region additionally will include the bacteriophage $O_L$ operator. The gene $C_I857$ which codes for the temperature-sensitive $C_I$ repressor will be included in the secretion expression vector. This will allow regulation of the promoter by interaction between the repressor and the operator at low temperatures (for example, about 30°C). Increasing the temperature to about 42°C will inactivate the repressor and allow expression of the gene of interest.

The transcriptional regulatory region optionally may include regulatory sequences which terminate transcription and which provide sequences or structures which inhibit degradation of the mRNA and thus increase the stability of the mRNA species and allow for higher expression. Several examples of prokaryotic sequences are known (for example, the Trp terminator, the gene 32(T4) terminator, or a synthetic terminator which is similar in sequence to gene 32).

Secretion of the polypeptide of interest can be achieved by using a signal sequence. The DNA sequence encoding the signal sequence is joined upstream from and in reading frame with a DNA sequence encoding a polypeptide of interest. Typically, the signal sequence includes a cleavage site which is recognized by a signal sequence peptidase. Thus, positioning the polypeptide of interest directly after the signal sequence cleavage site will allow it to be specifically cleaved from the signal sequence and secreted as a mature polypeptide.

The DNA sequence encoding the signal sequence may be any DNA sequence encoding a signal sequence obtainable from a gene encoding a polypeptide which is normally secreted. By "obtainable" is intended a DNA sequence which can be obtained from a natural source. The DNA sequence encoding the signal sequence may be identical to that of a naturally occurring sequence or may have changes in the sequence so long as the changes do not affect the ability of the resulting signal sequence to direct secretion of the polypeptide of interest. Examples of signal sequences include the signal sequences from the ompA gene (Movva et al., Journal of Biochemistry (1980) 255:27-29); the phoA gene (Inouye et al. Journal of Bacteriology (1982) 149:434-439); the ompF gene (Itebgbeph et al., Proc. Nat. Acad. Sci (USA) (1980) 77:2621-2625), the ompC gene (Mizuno, et al. FEBS Lett. (1983) 151:159-164), the phoE gene (Overbeck, et al., J. Mol. Biol. (1983) 163:513-532), the ompF gene (Mutuh FEBS Lett. (1982) 137:171-172).

Other signal sequences which may find use in the subject invention may be identified based upon the following characteristics. The signal sequences are generally approximately of the same length, namely about 20 to 30 amino acids. Additionally, the amino acid sequence of a signal sequence starts with a segment of about 1 to 7 hydrophilic amino acids, the segment being terminated by a short amino acid sequence containing about 1 to 3 positively charged amino acid residues. Generally, the remaining portion of a signal sequence is comprised of a hydrophobic region containing about 15 to 20 amino acid residues, commonly referred to as a hydrophobic core. Such sequences may be identified by, for example, computer scanning to determine the hydrophobicity curve for an amino acid sequence encoded by an open reading frame.

Generally the signal sequences will be prepared by synthesizing oligomers having the desired sequence as described above. In some instances, it may be necessary or desirable to introduce restriction sites into the DNA sequence encoding the signal sequence. Where possible, the changes are silent with respect to the amino acid sequence of a naturally occurring signal sequence. As necessary, restriction sites may be included at the 5'-end and/or the 3'-end of the synthetic signal sequence to facilitate its insertion into an appropriate expression plasmid in conjunction with a DNA sequence encoding a polypeptide of interest.

The polypeptide of interest may be any polypeptide for which expression is desired and may be a polypeptide which is either homologous (encoded by DNA derived from the host cell) or heterologous (encoded by DNA derived from a foreign source or a wholly or partially synthetic DNA sequence) to the host cell. Where the polypeptide is homologous to the host cell, it may be indigenous or endogenous to the host cell. The polypeptide may be derived from prokaryotic sources, or eukaryotic sources, which eukaryotic sources may include fungi, protists, vertebrates, invertebrates, and the like. The polypeptide of interest may include enzymes such as antitrypsin-$\alpha$ and DHFR; mammalian peptides, such as any of the interferons ($\alpha$, ß, $\gamma$), IL-2, and the like; or it may be a chimeric polypeptide, derived from more than one polypeptide.

Where the gene of interest is to be expressed in a host which recognizes the natural transcriptional and translational regulatory regions of the desired gene of interest, the entire gene with its natural 5' and 3' regulatory regions may be introduced into an appropriate secretion expression vector. However, where the gene is to be expressed in a host which does not recognize the natural transcriptional and translational regulatory regions associated with the gene of interest, further manipulation may be required. The noncoding or 5'-region upstream from the gene of interest may be removed by endonuclease restriction, Bal31 digestion, or the like. Alternatively, where a convenient restriction site is present near the 5'-terminus of the gene of interest, the gene of interest may be restricted and an adapter employed for linking the gene of interest to the promoter region, where the adapter provides for the lost nucleotides of the gene of interest. A variety of 3'-transcriptional regulatory regions are known and may be inserted downstream from the stop codons.

The DNA sequences encoding the polypeptide of interest can be synthesized using conventional techniques giving overlapping single strands which may be annealed to define the desired coding sequences. The termini can be designed to provide restriction sites, or one or both termini may be blunt-ended for ligation to complementary ends of an expression vector. Expression vectors are generally available and are amply described in the literature.

Instead of synthesizing the DNA encoding the polypeptide of interest, the DNA may be isolated by various techniques. These include isolating mRNA from a host organism which codes for the polypeptide of interest, the mRNA reverse-transcribed, the resulting single-stranded (ss) DNA used as a template to prepare double stranded (ds) DNA, and the ds DNA isolated. Another technique is to isolate a piece of the host cell genomic DNA and (using a probe, appropriately degenerate, comprising a region with the most conserved sequences of the gene encoding the polypeptide of interest) identify sequences encoding the polypeptide of interest in the host genome. The probe can be considerably shorter than the entire sequence, but should be at least 10, preferably at least 14, more preferably at least 20, nucleotides in length. Longer oligonucleotides are also useful, up to the full length of a gene encoding the polypeptide of interest. Both DNA and RNA probes can be used. Although probes are normally used with a detectable label that allows for easy identification, unlabeled oligonucleotides are also useful, both as precursors of labeled probes and for use in methods that provide for detection of DNA or DNA/RNA. Accordingly, the term oligonucleotide refers to both labeled and unlabeled forms.

Depending upon the source of the DNA sequence encoding the polypeptide of interest and the length of the desired polypeptide, convenient restriction sites may be designed into the DNA encoding the polypeptide of interest. When possible, the restriction site(s) is(are) silent with respect to the amino acid sequence, i.e., it leaves the amino acid sequence of the polypeptide unaltered (for example, changing a serine codon from AGC to AGT or an isoleucine codon from ATT to ATC). However, in some cases, incorporation of the new restriction site(s) may yield an altered amino acid sequence without substantially changing the activity of the protein.

During the construction of the secretion expression vectors, various fragments of the DNA will usually be cloned in an appropriate cloning vector, which allows for amplification of the DNA, modification of the DNA, or manipulation by joining or removing sequences, linkers or the like. Normally, the vectors will be capable of replication in at least a relatively high copy number in bacteria. A number of vectors are readily available for cloning in gram-negative bacteria, especially $E. \underline{coli}$, including such vectors as pBR322, pUC18, pUC19, SP6, and the like. The cloning vectors are characterized by having an efficient replication system functional in the host bacterium.

The cloning vector will have at least one unique restriction site, usually a plurality of unique restriction sites, and may also include multiple restriction sites. In addition, the cloning vector will have one or more markers which provide for selection of transformants. The markers will normally provide resistance to cytotoxic agents which may be antibiotics, heavy metals, toxins and the like; complementation of an auxotrophic host; or immunity to phage. By appropriate restriction of the vector and the cassette and, as appropriate, modification of the ends, by chewing back or filling in overhangs to provide for blunt ends, by additional linkers, and by tailing, complementary ends can be provided for ligation of the vector to the secretion expression vectors or component[s??] thereof.

After each manipulation of the DNA in the development of the cassette, the resulting plasmid will be cloned and isolated and, as required, the particular secretion vector component analyzed as to its sequence to ensure that proper sequence has been obtained. Depending upon the nature of the manipulation, the desired sequence may be excised from the plasmid and introduced into a different vector where the plasmid may be restricted and the secretion expression vectors component manipulated, as appropriate.

In some instances a shuttle vector will be employed where the vector is capable of replication in different hosts requiring different replication systems. This may or may not require additional markers which are functional in the two hosts. Where such markers are required, these can be included in the vector. The plasmid containing the vector, two replication systems and the marker(s) may be transferred from one host to another, as required. For selection, any useful marker may be used. Resistance to neomycin or tetracycline is of interest. However, although a marker for selection is highly desirable for convenience, other procedures for screening transformed cells have been described. See, for example, G. Reipin et al., Current Genetics (1982) 189-193. Transformed cells may also be screened by the specific products they make; for example, synthesis of a desired product may be determined by immunological or enzymatic methods.

Once the secretion vector comprising the DNA sequence encoding the polypeptide of interest has been introduced into an appropriate host, the host may be grown to express the DNA sequence encoding the polypeptide of interest. A variety of prokaryotic hosts may be employed. Host cells can include any gram-negative organisms that do not have a temperature-sensitive mutation, such as $E. \underline{coli}$, for example, JM107, JM105, JM101, or DH5α.

The host cell may be grown to high density in an appropriate nutrient medium. At the time it is desired to obtain expression of the DNA sequence encoding the polypeptide of interest, the temperature of the nutrient medium can be changed. For example, where the regulatory sequence comprises the bacteriophage $\lambda P_L$ promoter, the bacteriophage $O_L$ operator, and the $C_I857$ temperature-sensitive repressor, the host cells may be grown at a permissive temperature, generally about 30°C, at which temperature transcription from the $P_L$ promoter is repressed and the host cells may grow unhampered by the demands of the synthesis of a foreign gene product, which additionally may be toxic to the host organism. When the host cells have reached an appropriate density, the temperature may be increased to a nonpermissive temperature (for example, about 42°C) at which temperature the $C_I857$ repressor is rendered inactive, permitting transcription from the $P_L$ promoter.

Where the product is secreted into the periplasmic space, the cells are harvested and the product is liberated by destruction of the cell wall (for example, by hypotonic shock, and the like). Where the product is secreted into the medium, the nutrient medium may be collected and the product isolated by conventional means (for example, affinity chromatography). To produce an active protein it may be necessary to allow the protein to refold.

The following examples are offered by way of illustration and not by way of limitation.

## EXPERIMENTAL

### Enzymes and Reagents

Restriction enzymes were purchased from New England Biolabs, BRL, and IBI. Bacteriophage T4 DNA ligase and polynucleotide kinase were obtained from New England Biolabs. Calf intestinal phosphatase was obtained from Boehringer-Mannheim. $[^{35}S]$-methionine, $[\alpha\text{-}^{35}S]ATP$, $[^{32}P]ATP$ and $[^{14}C]$-methionine protein standards were purchased from Amersham. $[^{125}I]\text{-}\alpha$-bungarotoxin was purchased from New England Nuclear. Rainbow protein standards were obtained from Amersham. Acrylamide and urea were purchased from ICN. Agarose was purchased from Bio-Rad and FMC. Ten-twenty percent gradient SDS-PAGEs were obtained from ISS. Nitrocellulose filters and Elutip columns were purchased from Schleicher and Schuell. A DNA sequencing kit was purchased from US Biochemicals. X-ray film was obtained from Kodak, and CsCl and phenol were purchased from IBI. NenSorb columns were purchased from New England Nuclear.

### Bacterial Strains and Plasmids

E. coli K12 JM107 was the host strain for all procedures. The plasmid pUC18 was purchased from BRL. The plasmid pHE6 was a generous gift from G. Milman, Methods in Enzymology (1987) 153:482-491.

### Synthesis and Purification of Oligomers

Oligomers were synthesized using a Biosearch 8600 DNA Synthesizer and Biosearch reagents. Oligomers were gel-purified on 20% polyacrylamide-urea gels, desalted on NenSorb columns and lyophilized.

### Purification of Plasmid DNA and DNA Cloning

Alkaline lysis of cells was performed as previously described (Birnboim et al., Nucleic Acid Res. (1979) 7:1513-1523). Plasmid DNA was purified using a two-step CsCl gradient centrifugation method (Garger et al., Biochem. Biophys. Res. Commun. (1983) 117:835-842). Standard DNA cloning techniques were performed as previously described (Maniatis et al., A Laboratory Manual (1982)).

### DNA Sequencing

DNA sequencing was performed on alkaline-denatured plasmid DNA using the protocol supplied with the US Biochemical sequencing kit. Samples were electrophoresed on 8% polyacrylamide-urea gels using a BRL apparatus.

### Protein Sequencing

Protein sequencing was performed according to a method described by Speicher et al., Techniques in Protein Chemistry (1989) 24-25, using an Applied BioSystems 477-A protein sequencer and Applied BioSystems reagents.

### EXAMPLE 1

### Cloning of Synthetic Signal Sequences

Two different signal sequences, from the ompA gene (Movva et al., (1980) supra) or phoA gene (Inouye et al., (1982) supra) of E. coli were used in the construction of these vectors. The oligomers used to construct the synthetic ompA and phoA signal sequences are shown in Figure 1, A and B. The following changes, all silent with respect to the amino acid sequence, were made in the naturally occurring sequences. For ompA, the isoleucine codon ATT in position 16 was changed to ATC, creating new restriction sites for PvuI and NruI. For phoA, the initial GTG methionine codon was changed to ATG, and the serine codon at position 18 was changed from AGC to AGT, creating a new ScaI restriction site. A polylinker was included at the 3′-end of each signal sequence to facilitate the insertion of any gene of interest. The 5′-end of each sequence was designed so that the coding region, of the lambda N-protein gene (Franklin and Bennett, Gene (1979) 8:107-119) of pHE6 could be removed at the 7 HinfI restriction site and replaced by either signal sequence. The oligomer sequences from the HinfI site to the initial ATG codon are identical to those of the N-protein gene and maintain the natural ribosome binding site.

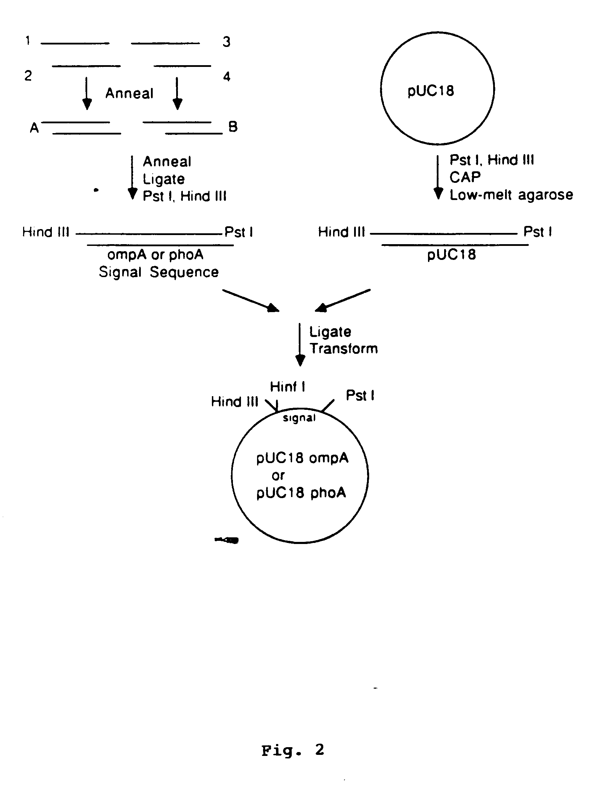

The diagram for cloning the signal sequences is shown in Figure 2. The following protocol was used for both ompA and phoA signal sequences. Kinased oligomers were mixed pairwise, 1+2 and 3+4, in equimolar amounts and lyophilized. After resuspending in 10 1 $H_2O$, the oligomer pairs were heated 5 minutes at 90°C and slowly cooled to 40°C. The resulting annealed oligomer pairs, A and B, were mixed together and incubated at 35°C for 60 minutes. A prewarmed mix of 10 µl 10 mM ATP and 39 µl $H_2O$ was added to the set of oligomers. The incubation temperature was lowered by 3°C every 5 minutes to 15°C. Ligase was added, and the oligomer set was incubated overnight at 15°C. After heating 10 minutes at 70°C to inactivate the ligase, the oligomer set was digested with HindIII and PstI restriction enzymes. The resulting 107 bp HindIII-PstI restriction fragment was ligated to appropriately prepared pUC18. Competent JM107 cells were transformed with the ligation mixture, and six transformants were selected for DNA sequence analysis. Plasmid DNA, pUC18ompA, from a transformant with no mutations in the synthetic ompA signal sequence, was used in subsequent constructions. This process was repeated for the phoA signal sequence, creating pUC18phoA.

EXAMPLE 2

Construction of pNB1 and pNB2

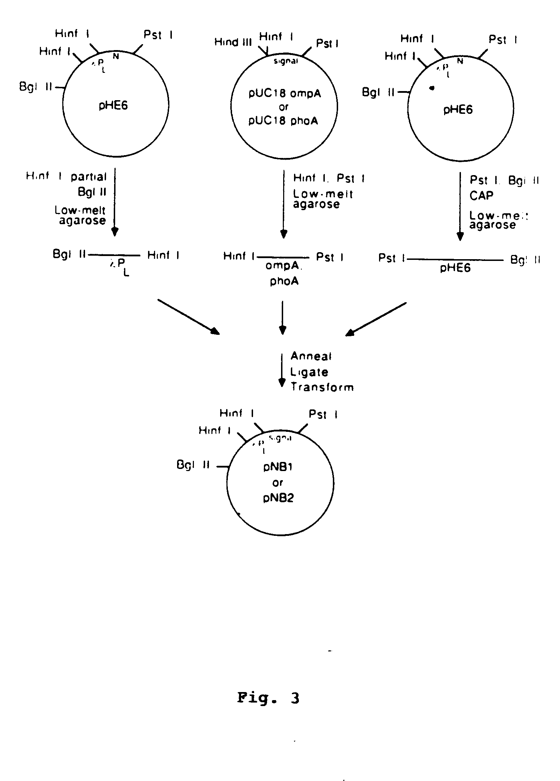

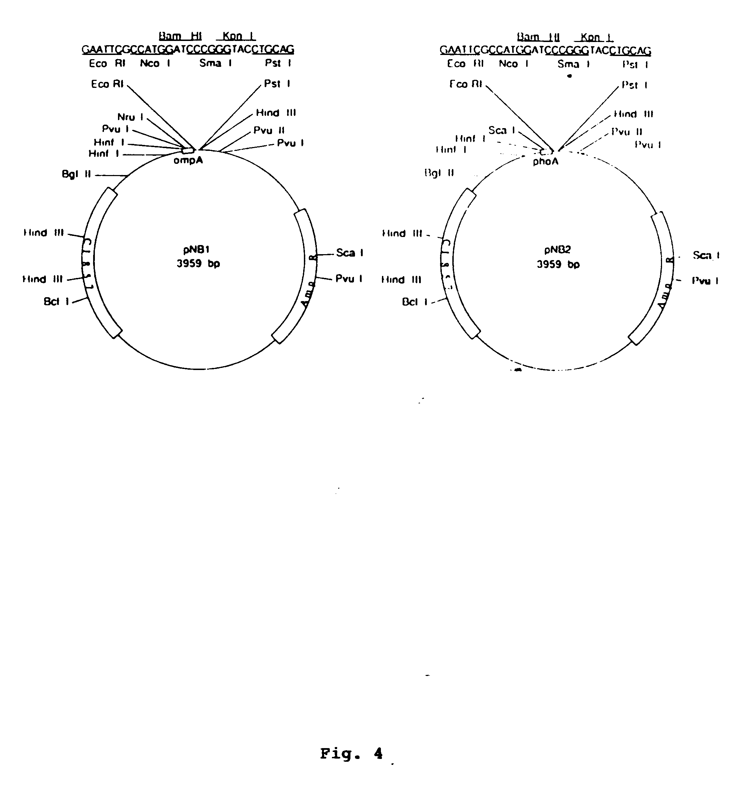

The diagram for the construction of pNB1 and pNB2 is shown in Figure 3. HinfI-PstI restriction fragments carrying the ompA and phoA synthetic signal sequences were generated from pUC18ompA and pUC18phoA, respectively. Ten µg of pHE6 were partially digested with HinfI to generate a majority of single-cut plasmid DNA. This DNA then was digested with BglII to yield a 348 bp HinfI-BglII restriction fragment carrying the lambda $P_L$ promoter ($\rho\gamma p_L$). Five µg each of pUC19ompA and pUC18phoA were digested with HinfI and PstI, generating 101 bp HinfI-BglII restriction fragments carrying the synthetic ompA and phoA signal sequences. The restriction fragments were gel-purified in 1.8% low-melt agarose, phenol-extracted and passed through a Schleicher and Schuell Elutip column according to manufacturer's directions. An additional microgram of pHE6 was digested with PstI and BglII, dephosphorylated with calf alkaline phosphatase (CAP), and similarly gel-purified in 0.8 low-melt agarose. The ompA and phoA HinfI-PstI restriction fragments were each mixed with the λP_L BglII-HinfI restriction fragment and the prepared pHE6 vector DNA. The DNA mixtures were heated 5 minutes at 70°C, slowly cooled to 16°C, and ligated overnight at 16°C. The ligated mixtures then were used to transform competent JM107 cells. Several transformants from each construction were selected for DNA sequence analysis to verify the correct assembly of the restriction fragments. Maps of the completed temperature-inducible secretion vectors pNB1 and pNB2 are shown in Figure 4.

EXAMPLE 3

Expression of the Gene Encoding the α Subunit of the Human Acetylcholine Receptor

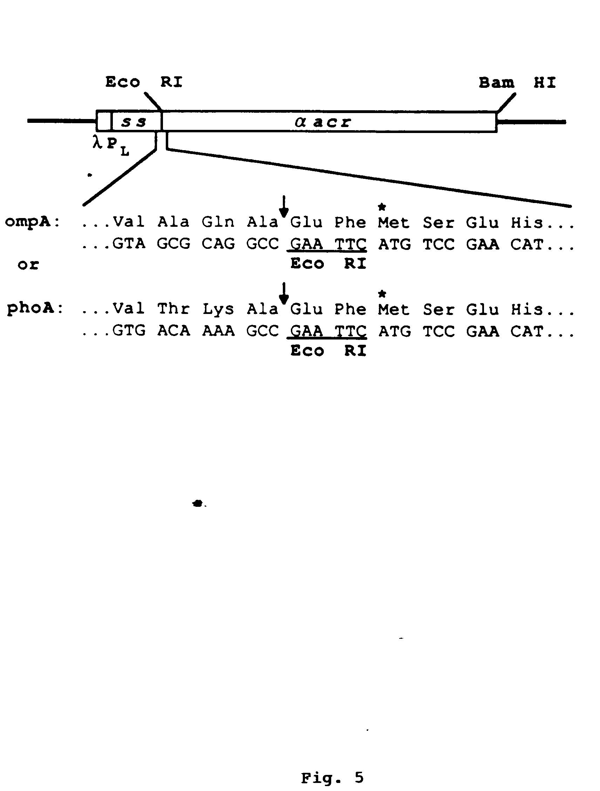

To determine if the expression vectors pNB1 and pNB2 function as designed, a synthetic gene encoding the theoretical amino terminal extracellular portion of the alpha subunit of the human acetylcholine receptor, αacr (see copending application filed (atty docket no. AISI-015)) was inserted between the EcoRI and BamHI restriction sites of the polylinker in each vector (Figure 5), creating pNB1αacr and pNBαacr. The plasmids pNB1 and pNB2 were digested with EcoRI followed by BamHI, dephosphorylated with CAP and gel-purified in 1.0% low-melt agarose. Each vector was mixed with a gel-purified EcoRI-BamHI restriction fragment carrying αacr and ligated. The resulting nucleotide sequence of the fusion and the corresponding amino acid sequence of the region of each construction are shown in the expanded area of the diagram. The arrows (Figure 5) indicate the cleavage sites of the signal peptides; the asterisks mark the start of the αacr portion of the protein. DNA sequencing was performed to verify that the αacr gene was inserted in phase with the signal sequence of each vector.

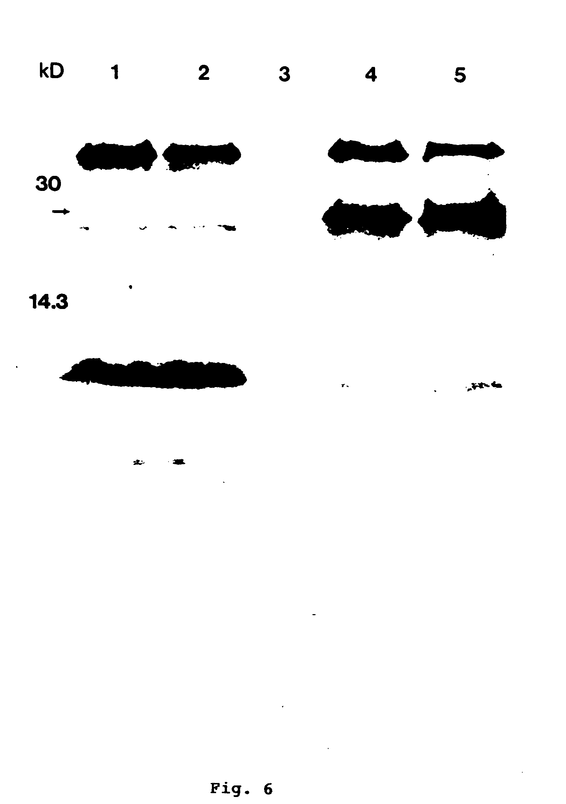

The plasmids pNB1αacr and pNB2αacr were tested initially for the ability to express the αacr protein in vitro with a DNA-dependent coupled transcription-translation system incorporating ³⁵S-methionine. Samples of each construction were used in conjunction with a kit purchased from Amersham. The manufacturer's recommended protocol for use with ³⁵S-methionine was followed. Samples were boiled 10 minutes, then electrophoresed on 10-20% SDS-PAGE. The gel was treated with Enlightening, purchased from NEN, dried and exposed to X-ray film for 10 minutes. The translation products were analyzed by SDS-PAGE, and the results are shown in Figure 6. Lane 1, pNB1; Lane 2, pNB2; Lane 4, pNB1αacr; Lane 5, pNB2αacr. Arrow indicates unique 28 kD proteins corresponding to ompAαacr and phoAαacr. Without the inserted gene, pNB1 and pNB2 each yielded a unique protein of approximately 7 kD (Lanes 1 and 2). These proteins are most likely run-on translation products from the signal sequence initiation to a random termination codon in the plasmid DNA 3′ of the polylinker region. With the αacr gene inserted into the polylinker of pNB1 and pNB2, the 7 kD protein was no longer produced. Instead, each vector yielded a unique protein of 28 kD (Lanes 4 and 5), corresponding to the expected molecular weight of the αacr protein plus the additional amino acids of either signal peptide, which would not be cleaved in vitro. The αacr protein was produced with equal efficiency from pNB-2αacr or pNB2αacr in vitro.

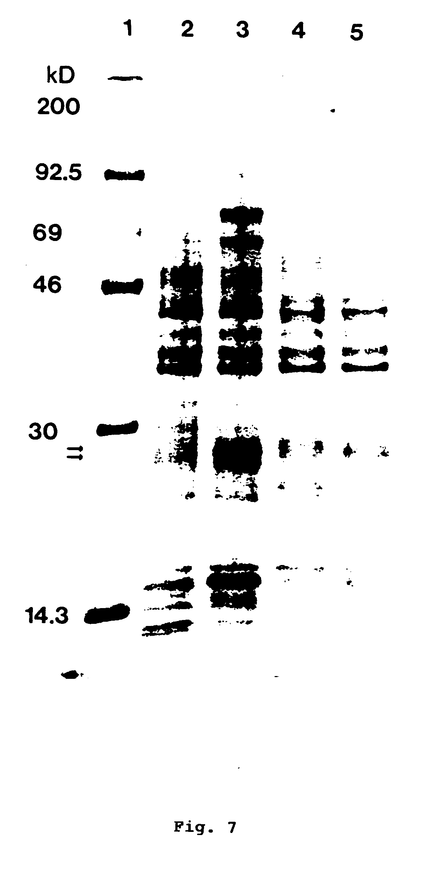

The expression vectors were tested next for the production of αacr protein in vivo. Cultures of JM107 harboring pNBαacr or pBN2αacr were grown in LB or M9CA at 30°C to $A_{600}$=0.6-0.8 O.D. For induction, cultures were rapidly transferred to 42°C for 45 minutes, then to 37°C for the remainder of the growth period. One-ml samples were removed three hours after induction. Cells were pelleted and resuspended in 20 µl protein sample buffer. After boiling for 10 minutes, 10 µl aliquots were electrophoresed on 10-20% SDS-PAGE and stained with Coomassie blue. The results are shown in Figure 7. Lane 1, protein standards; Lane 2, pNB1αacr-30°C; Lane 3, pNB1αacr-42°C; Lane 4, pNB2αacr-30°C; Lane 5, pNB2αacr-42°C.

Arrows indicate 28 kD and 25 kD proteins corresponding to ompAαacr and αacr, respectively. Two proteins of 28 kD and 24 kD appeared after induction (Lanes 2 and 3). The molecular weights of these proteins correspond to those expected for ompA-αacr (unprocessed) and αacr (processed).

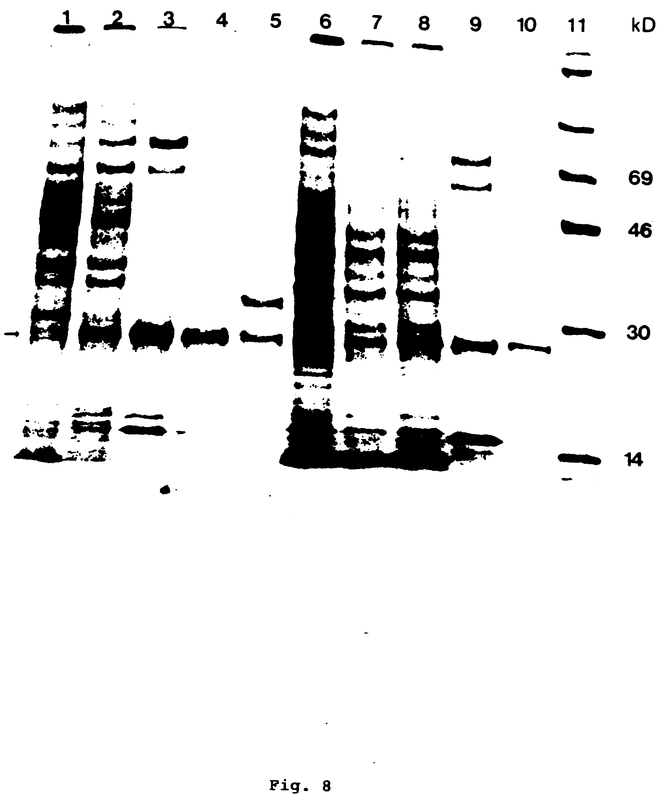

It was difficult to evaluate the expression of αacr protein in cells carrying pNB2αacr from a stained gel. Therefore, cultures carrying either plasmid were grown and induced as described, then pulsed with 35S-methionine at various timepoints after induction. Cultures of JM107 harboring either pNB1αacr or pNB-2αacr were grown and induced as described in Figure 7. At various timepoints after induction, 50 Ci of 35S-methionine were added to 1 ml aliquots of cells, and growth continued for 10 minutes. Total cell extracts were prepared and analyzed by SDS-PAGE followed by fluorography, as described in Figure 6. Samples from in vitro-coupled transcription-translation of pNB-1αacr and pNB2αacr were included for comparison. Lane 1, pNB1αacr-30°C; Lanes 2, 3 and 4, pNB1αacr-42°C, for 30 min., 1 and 2 hr, respectively; Lane 5, pNB1αacr-in vitro; Lane 6, pNB2αacr-30°C; Lanes 7, 8 and 9, pNB2αacr-42°C for 30 min, 1 and 2 hr, respectively; Lane 10, pNB2αacr-in vitro; Lane 11, 14C-methionine-labeled protein standards. Arrow indicates αacr proteins. Total cell extracts were analyzed by SDS-PAGE followed by fluorography. The results, shown in Figure 8, indicate that pNB-1αacr and pNB2αacr yielded roughly equivalent amounts of αacr protein in a 10-minute pulse. However, since cells carrying pNB1αacr appeared to accumulate much higher levels of αacr during growth after induction, the rest of this work focused on expression of αacr from pNB1αacr.

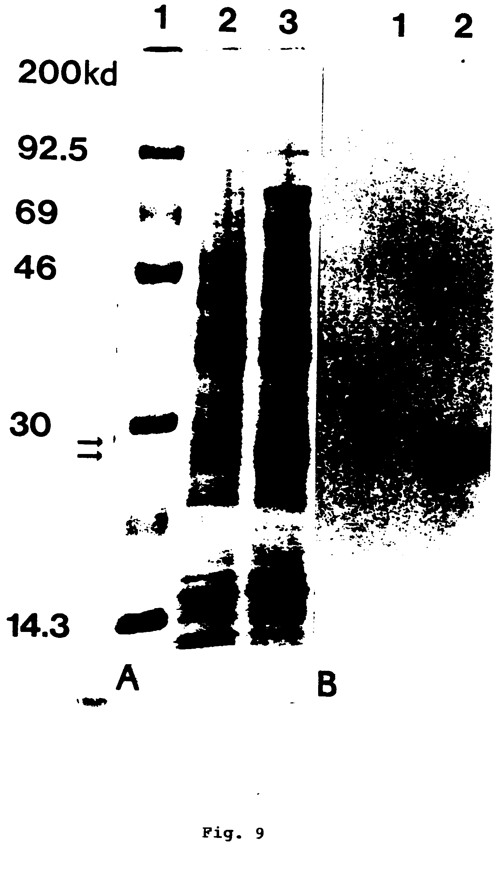

To confirm the identities of the 28 kD and 25 kD induced proteins as αacr proteins, and to evaluate their biological activities, an α-bungarotoxin binding assay was performed. This snake venom toxin binds specifically near the active site of the alpha subunit of acetylcholine receptors (Haggerty and Froehner, J. Biol. Chem. (1981) 256:8294-8297; Wilson et al., Proc. Natl Acad. Sci. (USA) (1985) 82:8790-8794). It also binds to the αacr protein expressed from pKK223-3 (see copending application USSN on even date herewith, attorney docket No. AISI-015). Total cell extracts from induced or uninduced cultures carrying pNB1αacr were subjected to SDS-PAGE and transferred to Blotto and incubated overnight at 4°C. The filter was transferred to fresh Blotto and incubated with 20 μCi 125I-α-bungarotoxin for 5 hours at room temperature. After washing 1 x 30 minutes in Blotto at 4°C and 3 x 15 minutes in TBS at 4°C, the filter was dried completely and exposed to X-ray film for 3 days at -70°C. The results are shown in Figure 9. Panel A: 10-20% SDS-PAGE stained with Coomassie blue. Lane 1, protein standards; Lane 2, pNB1αacr-30°C; Lane 3, pNB1-αacr-42°C. Panel B:

Autoradiogram of Western blot of an identical gel. Lane 2, pNB1αacr-42°C. The 125I-α-bungarotoxin bound specifically to the two induced proteins, confirming that these proteins are two forms of αacr and that each form has biological activity.

Preliminary data from microsequencing of the amino terminus of the 24 kD αacr protein indicates that correct processing of the signal peptide occurred. Cleavage occurred after the alanine residue in position 1, the last residue of the ompA signal peptide. The αacr protein retains the glutamic acid and phenylalanine residues coded by the linker sequence. This demonstrated that successful expression of a protein and subsequent cleavage of the signal peptide was obtained using the vector pNB1.

Together, the ompA-αacr and αacr proteins were estimated to be 5-10% of the total protein of induced cells. This is consistent with the levels of expression of other proteins seen previously with the λPL promoter (Milman, Methods in Enzymology (1987) 153:482-491; Piatak, J. Biol. Chem. (1988) 263:4837-4843). Comparison of the band intensities of ompA-αacr and αacr in a stained SDS-PAGE indicated that approximately half of the αacr protein expressed accumulated as the unprocessed precursor. Bolla et al. (Biotechnology (1987) 4:991-995) found an increase in precursor accumulation of ompA-hGH after IPTG induction. The accumulation of precursor appears to be a function of high-level expression of a protein and is not attributed to the use of the ompA signal sequence for secretion (Bolla et al., (1987) supra).

Other factors can influence the accumulation of unprocessed protein. Hussain et al., (1989) supra, reported that the extent of processing of ompA-ricin B chain was host-strain dependent. Ikemura et al., J. Biol. Chem. (1987) 262:7859-7864, reported that 50% of ompA-pro-subtilisin accumulated as unprocessed precursor after induction at 37°C, but that induction at a reduced temperature of 23°C resulted in a reduction of precursor accumulation. Therefore, the use of a more "permissive" host strain than JM107 and/or further reduction of the growth temperature from 37°C, after induction at 42°C, may improve overall yield of processed protein. Lowered growth temperature after induction may also improve yield by decreasing proteolytic activity in the periplasmic space (Oka et al., Proc. Natl Acad. Sci. USA (1985) 82:7212-7216), or a new periplasmic protease-deficient mutant of E. coli (Strauch and Beckwith, Proc. Natl Acad. Sci. USA (1988) 85:1576-1580) may be a more suitable host for the production of secreted protein.

Efforts to recover soluble αacr from the periplasmic space by osmotic shock (Neu and Heppel, J. Biol. Chem. (1965) 240:3685-3692) of induced cells harboring pNB1αacr were unsuccessful. SDS-PAGE analysis of the proteins located in the periplasmic, cytoplasmic and membrane fractions revealed that the αacr protein was associated with the membrane fraction. Two possible explanations could account for processed $\alpha$acr remaining with the membrane fraction. It is possible that the $\alpha$acr protein was only partially translocated across the inner membrane, even though the signal peptide is cleaved. Oshuye et al., Nucleic Acid Research (1983) 11:1283-1294, found that complete translocation of a protein after cleavage of the signal peptide was dependent upon the carboxyl terminus of that protein. However, such "jamming" in the cell membrane has been shown to be lethal to the cell (Bieker and Silhavy, Proc. Natl Acad. Sci. (USA) (1989) 86:968-972), and the induction of $\alpha$acr expression did not lead to cell death.

A more likely explanation is that the transported $\alpha$acr protein was insoluble in the periplasmic space. Although there have been numerous reports of obtaining soluble forms of foreign protein secreted into the periplasmic space via a signal peptide (Hsiung et al., (1986), supra; Quaas et al., European J. Biochem. (1988) 173:617-622; Piatak et al., (1988), supra; Ray et al., Biochem. (1985) 97:1492-1436), some secreted foreign proteins remain insoluble (Ikemura et al., (1987) supra) or are found in both soluble and insoluble forms (Hussain et al., (1989), supra) in the periplasmic space. The ability of a foreign protein to fold into a soluble form in the periplasmic space of E. coli appears to be a function of the particular protein in question.

EXAMPLE IV

Expression of SG[1] and SGB[2] Using the Temperature-Inducible Secretion Vectors pNB1 and pNB2

Expression of SG and SBG

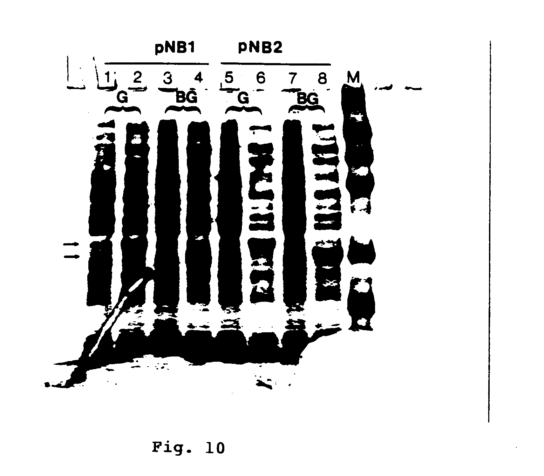

Synthetic protein G and chimeric B-G were cloned in the polylinker region of pNB1 and pNB2, and the resulting plasmids, pNB1-G, pNB1-BG, pNB2-G and pNB2-BG in E. coli, were grown at 30° to 0.6 O.D./600 nm, then induced at 42°C. Total cell extracts from these plasmids were analyzed by SDS-PAGE (Figure 10). The results show that both of the proteins SG and BG (Lanes 1, 4 and 6, 8) were expressed in expression plasmid pNB1 and pNB2. Uninduced samples served as controls (Lanes 1, 3, 5 and 7).

Localization of SG and SBG

To show the localization of expressed SG and SBG in E. coli, cells harboring pNB1-G, pNB1-BG, pNB2-G and pNB2-BG were grown at 30°C to A600=0.6-0.8, induced at 42°C and fractionated as described above.

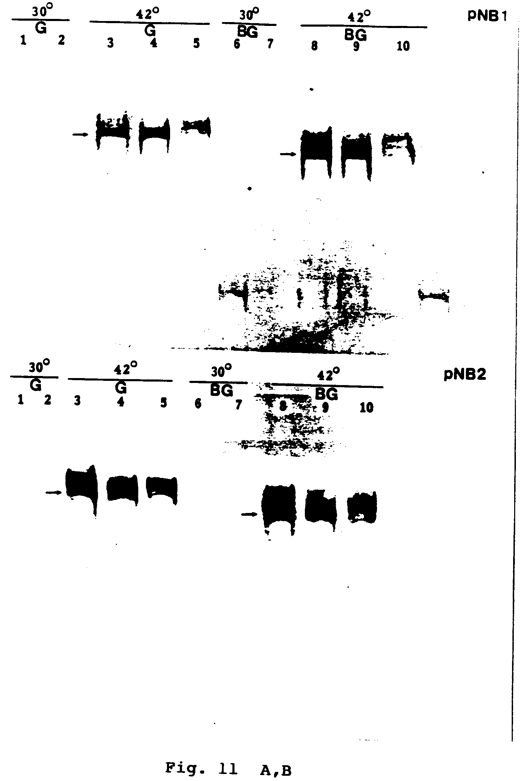

Periplasmic, spheroplast and total cell extracts were run on SDS-PAGE, transferred to nitrocellulose, incubated with human IgG and detected with Fab[26] HRP conjugate (Figure 2A and B). The results showed that the SG and chimeric BG proteins were synthesized, processed and secreted in soluble form into the periplasmic space. In Figure 11 is shown uninduced SG and SBG periplasmic and spheroplast fractions in Lanes 1, 2, 6 and 7, respectively. Induced SG or BG$_{periplasmic, spheroplast}$ and total cell extracts are shown in Lanes 3, 4, 5 and 8, 9, 10, respectively. Figure 11B shows similar results with pNB2-G or pNB2-BG.

These studies further validate that these expression plasmids (pNB1 and pNB2) are capable of transporting heterologous proteins into the periplasmic space.

Secretion expression vectors comprising a promoter which will bind to the C$_I$ repressor and a signal sequence have been constructed. The secretion vectors were used successfully to express heterologous protein in a microorganism. The protein was correctly processed and secreted into the periplasmic space, and shown to be biologically active. These temperature-inducible vectors present new alternatives for the high-level expression of foreign protein in prokaryotic cells.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

Claims

1. A secretion expression plasmid comprising: components joined in vitro including, in the 5'-3' direction of transcription, a transcriptional initiation region comprising a nucleotide sequence capable of binding to a C$_I$ repressor, a first DNA sequence encoding signal sequence capable of directing secretion of a polypeptide of interest from a prokaryotic cell, at least one restriction site for insertion of a second DNA sequence encoding a polypeptide of interest to be expressed under

[1]SG: A synthetic gene encoding IgG binding domains of streptocococcal protein G.
[2]SG: A Synthetic gene encoding a chimeric protein A (B-fragment) and the IgG binding domain of streptocococcal protein G.

said transcriptional initiation region and an optional transcriptional termination region, wherein said components are operably linked.

2. The secretion expression plasmid according to claim 1, wherein said transcriptional initiation region is the bacteriophage lambda $P_L$ promoter.

3. The secretion expression plasmid according to claim 1, wherein said first DNA sequence is obtainable from an ompA gene or a phoA gene.

4. A secretion expression vector comprising:

   components joined in vitro including, in the 5'-3' direction of transcription, a transcriptional initiation region comprising a nucleotide sequence capable of binding to a $C_I$ repressor, a first DNA sequence encoding a signal sequence capable of directing secretion of a polypeptide of interest from a prokaryotic cell, a second DNA sequence encoding a polypeptide of interest to be expressed under said transcriptional initiation region and an optional transcriptional termination region, wherein said components are operably linked.

5. The secretion expression vector according to claim 4, wherein said first DNA sequence is a synthetic sequence.

6. The secretion expression vector according to claim 5, wherein said synthetic sequence is at least substantially similar to an ompA or a phoA signal sequence.

7. A method for obtaining temperature-inducible secretion of a polypeptide of interest from prokaryotic host cells, said method comprising:

   growing in a nutrient medium at a permissive temperature prokaryotic host cells lacking a temperature-sensitive mutation and containing a secretion expression vector comprising components joined in vitro including, in the 5'-3' direction of transcription, a transcriptional initiation region comprising a nucleotide sequence capable of binding to a $C_I$ repressor, a first DNA sequence encoding a signal sequence, a second DNA sequence encoding a polypeptide of interest to be expressed under said transcriptional initiation region and an optional transcriptional termination region, wherein said components are operably linked, to obtain a pluiality of said host cells; and

   increasing the temperature of said nutrient medium to a temperature sufficient to induce transcription from said transcriptional initiation region ("induction temperature") whereby said polypeptide of interest is expressed and secreted from said host cells.

8. The method according to claim 7, wherein said prokaryotic host cells are E. coli.

9. The method according to claim 8, wherein said permissive temperature is about 30°C and 32°C and said induction temperature is about 42°C to 44°C.

10. The method according to claim 8, wherein said secretion is into the periplasmic space.

11. A prokaryotic cell comprising:

   a secretion expression vector comprising components joined in vitro including, in the 5'-3' direction of transcription, a transcriptional initiation region comprising a nucleotide sequence capable of binding to a $C_I$ repressor, a first DNA sequence encoding a signal sequence, a second DNA sequence encoding a polypeptide of interest to be expressed under said transcriptional initiation region and an optional transcriptional termination region, wherein said components are operably linked, wherein said cell lacks a temperature-sensitive mutation.

12. The cell according to claim 11, wherein said cell is an E. coli cell.

13. Plasmid pNB1 or pNB2.

**A)**  ompA  Oligomers

```
                        Met Lys Lys Thr Ala Ile Ala Ile Ala Val Ala Leu Ala Gly Phe Ala
(48) A GCT TGA ATC CAG ATG AAA AAG ACA GCG ATC GCG ATT GCA GTG GCA CTG GCT GGT TTC GCT
     (54) ACT TAG GTC TAC TTT TTC TGT CGC TAG CGC TAA CGT CAC CGT GAC CGA CCA AAG CGA

     Thr Val Ala Gln Ala Glu Phe Ala Met Asp Pro Gly Tyr Leu
     ACC GTA GCG CAG GCC GAA TTC GCC ATG GAT CCC GGG TAC CTG CA (57)
     TGG CAT CGC GTC CGG.CTT AAG CGG TAC CTA GGG CCC ATG G (43)
```

**B)**  phoA  Oligomers

```
                        Met Lys Gln Ser Thr Ile Ala Leu Ala Leu Leu Pro Leu
(54) A GCT TGA ATC CAG ATG AAA CAA AGT ACT ATT GCA CTG GCA CTC TTA CCG TTA
     (60) ACT TAG GTC TAC TTT GTT TCA TGA TAA CGT GAC CGT GAG AAT GGC AAT

     Leu Phe Thr Pro Val Thr Lys Ala Glu Phe Ala Met Asp Pro Gly Tyr Leu
     CTG TTT ACC CCT GTG ACA AAA GCC GAA TTC GCC ATG GAT CCC GGG TAC CTG CA (51)
     GAC AAA TGG GGA CAC TGT TTT CGG CTT AAG CGG TAC CTA GGG CCC ATG G (37)
```

FIG. 1

Fig. 2

Fig. 3

Fig. 4

Eco   RI                                                    Bam   HI

s s                          α a c r

λ P L

```
ompA:  ...Val Ala Gln Ala Glu Phe Met Ser Glu His...
       ...GTA GCG CAG GCC GAA TTC ATG TCC GAA CAT...
                              Eco   RI
  or
phoA:  ...Val Thr Lys Ala Glu Phe Met Ser Glu His...
       ...GTG ACA AAA GCC GAA TTC ATG TCC GAA CAT...
                              Eco   RI
```

Fig. 5

Fig. 6

Fig. 7

Fig. 8

Fig. 9

Fig. 10

Fig. 11  A,B